(19) Europäisches Patentamt
European Patent Office
Office européen des brevets

(11) EP 4 140 403 A1

# (12) EUROPEAN PATENT APPLICATION

(43) Date of publication:
01.03.2023 Bulletin 2023/09

(21) Application number: 21193169.6

(22) Date of filing: 26.08.2021

(51) International Patent Classification (IPC):
*A61B 5/024* (2006.01)     *A61B 5/00* (2006.01)

(52) Cooperative Patent Classification (CPC):
A61B 5/02411; A61B 5/4356; A61B 5/4362;
A61B 5/7267

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Nemo Healthcare B.V.
5504 DB Veldhoven (NL)

(72) Inventors:
• MELAET, Raoul
5504 DB Veldhoven (NL)
• KOK, René David
5504 DB Veldhoven (NL)
• VULLINGS, Rik
5504 DB Veldhoven (NL)

(74) Representative: DeltaPatents B.V.
Fellenoord 370
5611 ZL Eindhoven (NL)

## (54) FETAL HEART RATE SIGNAL PROCESSING

(57) Some embodiments are directed to a device (301) for processing a fetal heart rate signal. The device obtains a first fetal heart rate segment and a subsequent second fetal heart rate segment of a fetal heart rate signal, and a first uterine activity segment and a second uterine activity segment of a uterine activity signal, corresponding respectively to the first and second fetal heart rate segments. A fetal heart rate prediction model is applied to representations of the first fetal heart rate segment, the first uterine activity segment, and the second uterine activity segment, to obtain a predicted representation of the second fetal heart rate segment. The predicted representation is compared to the obtained second fetal heart rate segment to determine an anomaly score indicative of fetal health.

Fig. 3b

EP 4 140 403 A1

**Description**

**FIELD OF THE INVENTION**

The invention relates to a device for processing a fetal heart rate signal, and to a corresponding computer-implemented method. The invention further relates to a device for training a fetal heart rate anomaly detection, and to a corresponding computer-implemented method. The invention further relates to a computer readable medium.

**BACKGROUND**

The health of a fetus is strongly influenced by the supply of oxygen to the brain and organs. Especially during birth, hypoxia (i.e. lack of sufficient oxygen in the fetal tissues) can lead to irreversible damage or even be life-threatening to the fetus. This requires robust and reliable techniques to monitor fetal distress in real-time. Once an anomalous situation is detected, the obstetrician should be notified, such that permanent damage can be prevented.

Currently, cardiotocography (CTG) is the worldwide standard for measuring fetal well-being during labour. CTG is a temporal recording of the fetal heart rate (FHR) and uterine contractions (UC). In clinical practice, the CTG is assessed overtime by human visual inspection. Accurate assessment of fetal health by human visual inspection is however hampered by poor specificity of the interpretation and high inter- and intra-observer variation. Moreover, continuous CTG monitoring has led to an increase in caesarean sections and instrumental vaginal births.

To overcome the limitations of visual inspection, computer-aided CTG methods are proposed as a way of assisting clinicians and warning them when to pay closer attention to a fetus and potentially intervene. It is known to apply signal processing techniques to objectively interpret CTG. More recently, it was proposed to use machine learning techniques to automatically assess temporal segments of CTG data. The paper "Classification of normal and hypoxic fetuses from systems modeling of intrapartum cardiotocography" by P. Warrick et al., IEEE Transactions on Biomedical Engineering, 57(4):771-779, 2010, proposes to use a support vector machine classifier to classify normal and hypoxic fetuses using CTG data. Half of the hypoxic cases were detected, with a false positive rate of 7.5% and an AUC score of 0.140, 1h 40 min before delivery.

**SUMMARY OF THE INVENTION**

Existing techniques for interpreting CTGs suffer from a number of disadvantages. Existing techniques provide promising results on the datasets that they are trained and validated on, but fail to reproduce these results on larger, heterogeneous datasets. Another disadvantage is the limited transparency and interpretability provided by existing techniques. Many machine learning models are more or less black boxes, which do not explain predictions in a way that they are easily interpretable by humans. This can be problematic, especially when the stakes are high, and mispredictions can have severe consequences. These disadvantages hamper the evolution of these known techniques into clinical practice.

In accordance with a first aspect of the invention, a device and a corresponding computer-implemented method are provided for processing a fetal heart rate signal, as defined by claims 1 and 13, respectively. In accordance with a further aspect of the invention, a device and a corresponding computer-implemented method are provided for training a fetal heart rate anomaly detection, as defined by claims 9 and 14, respectively. In accordance with an aspect of the invention, a computer-readable medium is provided as defined by claim 15.

In various aspects, an anomaly score is determined for a fetal heart rate segment. During labour, uterine contraction can act as an external stimulus to the fetus, evoking a response of the fetus that is reflected in the fetal heart rate. Abnormal responses of the fetal heart rate to uterine contractions may suggest a compromised condition of the fetus. Interestingly, to perform anomaly detection, the inventors envisaged to use machine learning to let a trainable model learn to predict normal responses of the fetal heart rate to uterine contractions. By comparing the actual response to the predicted normal response, an anomaly score may be determined that is indicative of whether the fetus shows an abnormal reaction of the fetal heart rate to uterine activity, and thereby indicative of fetal health.

Specifically, two subsequent time segments of a uterine activity signal and a corresponding fetal heart rate signal during labour may be measured. Conceptually, the anomaly detection may involve predicting what the fetal heart rate of a healthy fetus would look like in the second time segment, given the fetal heart rate and uterine activity recorded in the first time segment, and given the presence or absence of uterine contractions in the second time segment. Deviations between the predicted fetal heart rate and the recorded fetal heart rate may suggest that the fetal response to the uterine contractions is not normal and the fetal condition might be compromised. Accordingly, a trained fetal heart rate prediction model may be applied to a representation of the first fetal heart rate segment, a representation of the first uterine activity segment, and a representation of the second uterine activity segment to obtain a predicted representation of the second fetal heart rate segment. The representations can be the signal time segments themselves, or can be determined using encoder models as described herein, for example. The anomaly score may be determined by comparing the predicted representation of the second fetal heart rate segment to a representation of the second fetal heart rate segment that was actually measured, for example, an encoding by a fetal heart rate encoder model.

The fetal heart rate prediction model is preferably trained on data of healthy fetuses only. Consequently, the prediction model may learn to predict representations of fetal heart segments for healthy fetuses, in particular its response to uterine activity. The actual fetal heart rate response of the fetus can then be compared with the predicted response. When the two representations are similar, it is likely the fetus comes from the healthy population. If the representations show a significant difference, this can indicate that the condition of the fetus is compromised, so the clinician can be warned for example to take additional diagnostic or intervention action. In other words, the anomaly score is determined based on the expectation that the FHR prediction is less accurate in case the health of the fetus is compromised and the fetus no longer responds in the same way as a healthy fetus would do. In particular, in an embodiment, a device as proposed herein may be configured to raise an alert if the anomaly score and/or an aggregate overall anomaly score exceeds a threshold.

The proposed approach has a number of advantages. The technique can be applied non-invasively, e.g., no pH measurements are needed, and it allows automated continuous monitoring to take place, reducing the risk that abnormalities and associated health risks during labour go undetected. The provided techniques can be applied in a hospital, but also at home. The equipment needed is relatively simple and easily transportable.

The approach is found to provide good performance in detecting fetuses at risk. The approach is also interpretable, because it is based on physiological principles since it essentially models the fetal response to uterine contractions. In particular, a prediction of the second fetal heart rate segment may be presented to a clinician, for example in combination with the segment that was actually measured and the corresponding uterine activity time segments, thus allowing to present the outcome of the anomaly detection in a representation that the clinician can easily understand and interpret.

Another important advantage is that the training can be performed using just training data of healthy fetuses. This making data collection much easier, since unhealthy fetuses are relatively rare and it is difficult to obtain a representative training dataset of unhealthy cases, especially since such cases of course in practice often lead to an intervention taking place, so that it is unknown what the final outcome without the intervention would have been. Since data of healthy fetuses only is used, it is easier to collect sufficient data to obtain a general model, or to adapt the model to different situations.

Accordingly, techniques are provided to process fetal heart rate and uterine activity signals (e.g., combined in a CTG), and to detect anomalies in fetal health. For example, an alert may be raised if an anomaly is detected, in other words, if the fetus shows an abnormal reaction of the fetal heart rate to the uterine activity, indicating that fetal health may be compromised. The anomaly score or the alert by itself does not provide attribution to a particular clinical picture, only a deviation from the normal situation. Such a deviation can have many different causes. Thus, the anomaly score itself may not provide a diagnosis but rather raise an alert that can trigger a clinician, e.g., a gynaecologist, to perform follow-up research, e.g., perform invasive pH measurements, and/or perform an intervention. By performing the anomaly detection, e.g., by continuously monitoring the fetal health, there is less risk of fetal health problems being missed.

A uterine activity signal is also known as a uterine contraction signal, and may be measured as an amount of pressure, e.g., expressed in mmHg or similar or as a percentage. The fetal heart rate may be measured in terms of a frequency, e.g., beats per minute.

In an embodiment, the fetal heart rate signal and/or the uterine activity signal may be obtained by electrophysiological measurements, e.g., electrocardiography for the fetal heart and/or electrohysterography for the uterine activity, from multiple electrodes on the abdomen of a pregnant woman. This way, the signals can be measured non-invasively, safely, and with relatively little discomfort for the pregnant woman. Interestingly, it is still possible to obtain sufficiently accurate signals to perform meaningful anomaly detection. Various alternatives are possible, e.g., instead of or in addition to electrodes, an external tocodynamometer may be used to obtain the uterine activity signal.

In an embodiment, in order to perform the prediction and/or the comparison, encodings of the fetal heart rate and uterine activity signals determined by respective trained encoder models may be used as representations. The encodings of the signals are typically lower-dimensional than the original signal themselves, thus essentially providing a compressed representation trained to extract the most relevant aspects of the signal. For example, respective autoencoders may be trained for the fetal heart rate and uterine activity signals, and the latent space representations determined by the autoencoder may be used as encodings. The prediction of the fetal heart rate may be performed in terms of such encodings by means of a fetal heart rate prediction model that is trained to predict an encoding of a second fetal heart rate segment, based on the encodings of the first fetal heart rate segment, the first uterine activity segment, and the second uterine activity segment.

Interestingly, the comparison between the predicted representation of the second fetal heart rate segment and the measured second feal heart rate segment can be performed in terms of encodings, without reconstructing a predicted second feal heart rate segment. Namely, the encoder model may be applied to the second fetal heart rate segment, and the representation obtained in this way may be compared to the predicted representation. Since the representations effectively select the most meaningful parts of the representations, this way of comparing the prediction to the measurement is particularly accurate.

Conceptually, the encodings may be regarded as modelling mechanisms in the body that regulate the heartrate in response to stimuli. The use of trained encoder models was found to result in representations for which an accurate prediction model can be trained that can be effectively used to detect anomalies. The use of encodings is also advantageous from an interpretability perspective. Namely, the predicted encoding of the second fetal heart rate segment can be used to understand why an anomaly was detected. For example, as is known per se, particular latent features of an encoding as produced by an autoencoder or similar feature extractor, may be identified to correspond to particular fetal behaviour. As another example, the encodings may be used to find similar cases that can be presented to the clinician. In an embodiment, a trained fetal heart rate decoder model corresponding to the encoder model may be applied to the predicted representation of the second fetal heart rate segment to obtain a predicted second fetal heart rate segment that can be output, e.g., along with the measured second fetal hart rate segment, e.g., for comparison by a clinician.

In an embodiment, the uterine activity encoder may determine a subsegment-by-subsegment encoding of subsegments of a uterine activity segment. Instead or in addition, the fetal heart rate encoder may determine a subsegment-by-subsegment encoding of a fetal heart rate segment. Such segmentwise encoders are relatively easy to train, e.g., independently of the prediction model, and can provide a good separation of concerns between the activities of the encoders and the prediction model. On the one hand, the encoders may be trained to efficiently represent the signals. On the other hand, the prediction model may be trained to perform an analysis of temporal aspects and/or relations between the different signals.

In an embodiment, the representation of the fetal heart rate segment determined by the fetal heart rate encoder model may comprise parameters of a probability distribution of a latent representation of the fetal heart rate segment. To determine the anomaly score, a divergence may be determined between the parameters of the probability distributions of the representations of the obtained and predicted second fetal heart rate segments, for example, in a segment-by-segment fashion.

In an embodiment, anomaly scores may be determined at multiple points in time and aggregated into an overall anomaly score. This way, for example, a clinician can get a first impression of the fetal health situation as it develops overtime. Various ways of aggregating the anomaly scores are possible as described herein. In an embodiment, an alert may be raised if the anomaly score and/or the overall anomaly score exceeds a threshold. This way, if an unexpected situation occurs, a clinician can be made aware of the situation so that appropriate action can be taken.

In an embodiment, the fetal heart rate prediction model is applied by applying a causal convolutional layer followed by one or more residual blocks. A model of this structure was found in practice to provide good results. In particular, the causal convolutional layer enables to represent temporal aspects of the signals, whereas the residual blocks allow to effectively move from a feature-based representation of the sensor signals towards the prediction. Skip connections in the residual blocks can improve training performance.

In an embodiment, a uterine activity encoder model may be used that is trained as a uterine activity autoencoder together with a corresponding uterine activity decoder model. Instead or in addition, a fetal heart rate encoder model may be used that is trained as a fetal heart rate autoencoder together with a corresponding a fetal heart rate decoder model. For example, either or both autoencoders may be variational autoencoders. By using autoencoders, interestingly, the encoder models can be trained to efficiently represent the underlying signals, and the training can be done separately of the training of the prediction model. Moreover, the decoder part of the autoencoder can be used to obtain a predicted heart rate segment from the encoding predicted by the prediction model.

In an embodiment, to train the fetal heart rate prediction model, the fetal heart rate prediction model may be applied to obtain a predicted representation of the second fetal heart rate segment. The fetal heart rate decoder model may be applied to the predicted encoding to obtain a predicted fetal heart rate segment; and the predicted fetal heart rate segment may be compared to the corresponding fetal heart rate segment of the training dataset. Accordingly, during the training, the predicted heart rate segment may be compared to the heart rate segment that was actually measured, thus encouraging that the representation provided by the prediction model matches reality. Instead of comparing the heart rate segments directly, it is also possible to compare the heart rate segments in encoded form, however.

In an embodiment, when using the fetal heart rate decoder model to obtain a predicted fetal heart rate segment, the decoder model may be applied in a segment-by-segment fashion by applying the fetal heart rate decoder model to respective subsegments of the predicted encoding to obtain respective subsegments of the fetal heart rate segment. The respective subsegments may be concatenated. To obtain the predicted fetal heart rate segment, optionally, a subsegment transition smoothening operation may be applied to improve the alignment between subsequent segments. This smoothened predicted segment may then be compared to the actual segment. The smoothening may also be applied in use for example on the predicted fetal heart rate segment shown to a clinician. By using the smoothening also during training, the prediction model is encouraged to make encoded predictions that combine well with the smoothening.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

## BRIEF DESCRIPTION OF THE DRAWINGS

Further details, aspects, and embodiments of the invention will be described, by way of example only, with reference to the drawings. Elements in the figures are illustrated for simplicity and clarity and have not necessarily been drawn to scale. In the Figures, elements which correspond to elements already described may have the same reference numerals. In the drawings,

Fig. 1a schematically shows an example of an embodiment of a device for processing a fetal heart rate signal,
Fig. 1b schematically shows an example of an embodiment of a device for training a fetal heart rate anomaly detection,
Fig. 1c schematically shows an example of an embodiment of a storage for storing model data,
Fig. 2a schematically shows an example of an embodiment of a device for training a fetal heart rate anomaly detection,
Fig. 2b schematically shows an example of an embodiment of a device for training a fetal heart rate anomaly detection,
Fig. 2c schematically shows an example of an embodiment of a device for training a fetal heart rate anomaly detection,
Fig. 2d schematically shows an example of an embodiment of a device for training a fetal heart rate anomaly detection,
Fig. 2e schematically shows an example of an embodiment of a device for training a fetal heart rate anomaly detection,
Fig. 3a schematically shows an example of an embodiment of a device for processing a fetal heart rate signal,
Fig. 3b schematically shows an example of an embodiment of a device for processing a fetal heart rate signal,
Fig. 3c shows an example of a predicted fetal heart rate segment,
Fig. 3d shows an example of an anomaly detection of a fetal heart rate segment,
Fig. 4 schematically shows an example of an embodiment of a computer-implemented method of processing a fetal heart rate signal;
Fig. 5 schematically shows an example of an embodiment of a method of training a fetal heart rate anomaly detection,
Fig. 6 schematically shows a computer readable medium having a writable part comprising a computer program according to an embodiment,
Fig. 7 schematically shows a representation of a processor system according to an embodiment.

List of Reference Numerals:

| 100 | a device for processing a fetal heart rate signal |
| 101 | a device for training a fetal heart rate anomaly detection |
| 110 | a sensor interface |
| 120,121 | a data interface |
| 130,131 | a processor subsystem |
| 129 | a storage |

| 201-205 | a device for training a fetal heart rate anomaly detection |
| 229 | a storage |
| 221,221',222 | a uterine activity segment |
| 230 | a uterine activity encoder model |
| 231,232 | an encoding of a uterine activity segment |
| 239 | a uterine activity decoder model |
| 241,241',242 | a fetal heart rate segment |
| 250 | a fetal heart rate encoder model |
| 251,252' | an encoding of a fetal heart rate segment |
| 259 | a fetal heart rate decoder model |
| 260 | a fetal heart rate prediction model |
| 261 | a causal convolutional layer |

**EP 4 140 403 A1**

| 262 | a residual block |
| 263 | a dilated convolutional layer |
| 264 | a tanh activation |
| 265 | a sigmoid activation |
| 266 | a combination |
| 267 | a 1x1 convolutional layer |
| 268 | a skip connection |
| 268' | a final block output |
| 271-273 | a training unit |

| 300-301 | a device for processing a fetal heart rate signal |
| 310 | a sensor interface |
| 311 | electrodes |
| 329 | a storage |
| 321,322 | a uterine activity segment |
| 330 | a uterine activity encoder model |
| 331,232 | an encoding of a uterine activity segment |
| 341,342 | a fetal heart rate segment |
| 350 | a fetal heart rate encoder model |
| 351,352,352' | an encoding of a fetal heart rate segment |
| 360 | a fetal heart rate prediction model |
| 370 | an anomaly scoring unit |
| 371 | an anomaly score |

| 391-393, 398 | fetal heart rate signals |
| 394, 395, 397 | uterine activity signals |
| 396 | anomaly scores |
| 396-1, 396-2 | anomalous region |

| 1000 | a computer readable medium |
| 1010 | a writable part |
| 1020 | a computer program |
| 1110 | integrated circuit(s) |
| 1120 | a processing unit |
| 1122 | a memory |
| 1124 | a dedicated integrated circuit |
| 1126 | a communication element |
| 1130 | an interconnect |
| 1140 | a processor system |

## DETAILED DESCRIPTION OF EMBODIMENTS

While this invention is susceptible of embodiment in many different forms, there are shown in the drawings and will herein be described in detail one or more specific embodiments, with the understanding that the present disclosure is to be considered as exemplary of the principles of the invention and not intended to limit the invention to the specific embodiments shown and described.

In the following, for the sake of understanding, elements of embodiments are described in operation. However, it will be apparent that the respective elements are arranged to perform the functions being described as performed by them.

Further, the invention is not limited to the embodiments, and the invention lies in each and every novel feature or combination of features described herein or recited in mutually different dependent claims.

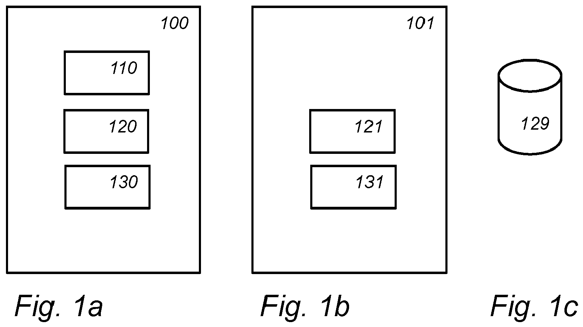

**Fig. 1a** schematically shows an example of an embodiment of a device for processing a fetal heart rate signal. **Fig. 1b** schematically shows an example of an embodiment of a device for training a fetal heart rate anomaly detection. **Fig. 1c** schematically shows an example of an embodiment of a storage for storing model data for use in a fetal heart rate anomaly detection, e.g., trained parameters of a fetal heart rate prediction model, a fetal heart rate encoder model, and/or a uterine activity encoder model.

Device 100 comprises a sensor interface 110, a data interface 120, and a processor subsystem 130. Device 101 comprises a data interface 121 and a processor subsystem 131.

The sensor interface 110 may be for accessing a fetal heart rate signal of a fetus and a corresponding uterine activity signal. Sensor interface 110 may be configured to obtain the fetal heart rate signal and/or the uterine activity signal from multiple electrodes. The multiple electrodes may be configured for arranging on the abdomen of a pregnant woman. For example, during use multiple electrodes are arranged on the abdomen of a pregnant woman during childbirth. The number of multiple electrodes may be, say, three or more, four or more, six or more, etc. Preferably, the electrodes are applied in a fixed arrangement. For example, the electrodes may be connected with each other using half-flexible connections so as to indicate the desired distance between electrodes. For example, ECG signals may be continuously recorded by device 100 during childbirth. The fetal heart rate signal and/or the uterine activity signal may be extracted from readings of the electrodes using techniques that are known per se, which may involve filtering out the mother's ECG signal to obtain the fetal ECG, and/or detecting QRS complexes.

Instead of or in addition to using the electrodes, various other options are possible. Uterine activity can for example be measured by performing electrophysiological measurements, e.g., using electrohysterography, by using a tocodynamometer, or an intra-uterine pressure catheter (IUPC). The fetal heart rate can be measured by performing electrophysiological measurements for example using electrocardiography. Alternatives for measuring the fetal heart rate include Doppler ultrasound, using an invasive fetal scalp electrode (FSE), using phonocardiography, ballistocardiography or seismocardiography. The electrodes and/or other measurement devices may be part of the device 100 or may be external to it.

The data interface 120 may be for accessing model data representing a fetal heart rate prediction model. The fetal heart rate prediction model may be configured to, based on a representation of a first fetal heart rate segment, a representation of a first uterine activity segment, and a representation of a second uterine activity segment, predict a representation of a second fetal heart rate segment. The model data may further represent a fetal heart rate encoder model, a fetal heart rate decoder model, a uterine activity signal encoder model, and/or a uterine activity decoder model. The model data may comprise trained parameters of the model(s), e.g., trained by device 101 or according to a method described herein. For example, the number of trained parameters of the model data may be at most or at least 1000, at most or at least 10000, or at most or at least 100000.

For example, the data interface 120 may be constituted by a data storage interface 120 which may access the model data from a data storage 129. For example, the data storage interface 120 may be a memory interface or a persistent storage interface, e.g., a hard disk or an SSD interface, but also a personal, local or wide area network interface such as a Bluetooth, Zigbee or Wi-Fi interface or an ethernet or fibreoptic interface. The data storage 129 may be an internal data storage of the system 100, such as a hard drive or SSD, but also an external data storage, e.g., a network-accessible data storage. In some embodiments, the data may each be accessed from a different data storage, e.g., via a different subsystem of the data storage interface 120. Each subsystem may be of a type as is described above for the data storage interface 120.

The processor subsystem 130 may be configured to obtain, via the sensor interface 110, a first fetal heart rate segment and a subsequent second fetal heart rate segment of the fetal heart rate signal. The processor subsystem 130 may be further configured to obtain, via the sensor interface 110, a first uterine activity segment and a second uterine activity segment of the uterine activity signal, corresponding respectively to the first and second fetal heart rate segments. The processor subsystem 130 may be further configured to apply the fetal heart rate prediction model to a representation of the first fetal heart rate segment, a representation of the first uterine activity segment, and a representation of the second uterine activity segment to obtain a predicted representation of the second fetal heart rate segment. The processor subsystem 130 may be further configured to determine an anomaly score indicative of fetal health by comparing the predicted representation of the second fetal heart rate segment to the obtained second fetal heart rate segment, and output the anomaly score.

The device 100 may further comprise an output interface (not shown) for outputting the anomaly score. For example, the output interface may be configured to report the anomaly score to a user, e.g., on a display or other output device connectable to device 100. For example, the output interface may be used to raise an alert if a determined anomaly score and/or overall anomaly score exceeds a threshold, e.g., on a display or in another sensory perceptible manner, e.g., as an audio signal. In some embodiments, the output interface may be separate from the data storage interface 120, but may in general be of a type as described above for the data storage interface 120.

The data interface 121 may be as described for data interface 120 of device 100. The data interface 121 may be for accessing model data as described above. The model data may be trained by device 101 for use by device 100. The data interface 121 may further be for accessing a training dataset comprising multiple fetal heart rate signals of fetuses and corresponding uterine activity signals. The training dataset may comprise at most or at least 1000 or at most or at least 10000 CTG recordings comprising the fetal heart rate and uterine activity signals, for example.

The processor subsystem 131 may be configured to, using the fetal heart rate signals and corresponding uterine activity signals of the training dataset, train a fetal heart rate prediction model to, based on a representation of a first fetal heart rate segment, a representation of a corresponding first uterine activity segment, and a representation of a subsequent second uterine activity segment, predict a representation of a second fetal heart rate segment. The processor subsystem 131 may be further configured to output the fetal heart rate prediction model, for example, by storing it on a storge accessible to device 100, e.g., a network storage, or for later provisioning to a device 100.

Devices 100 and 101 may be combined: the same device that trains the fetal heart rate prediction model and possibly also encoder/decoder models, may also determine anomaly scores for incoming signals using the trained model(s).

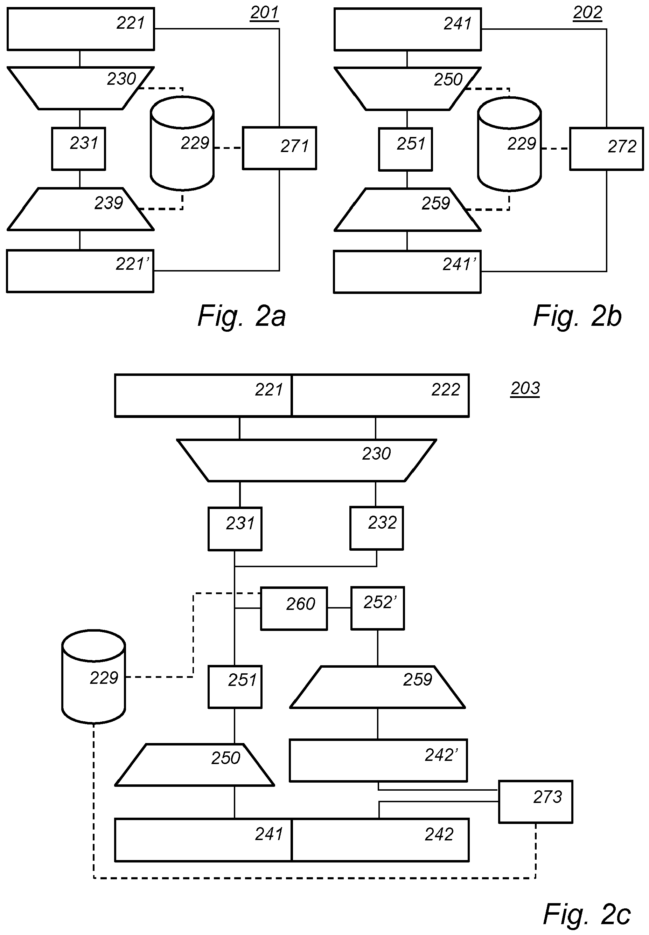

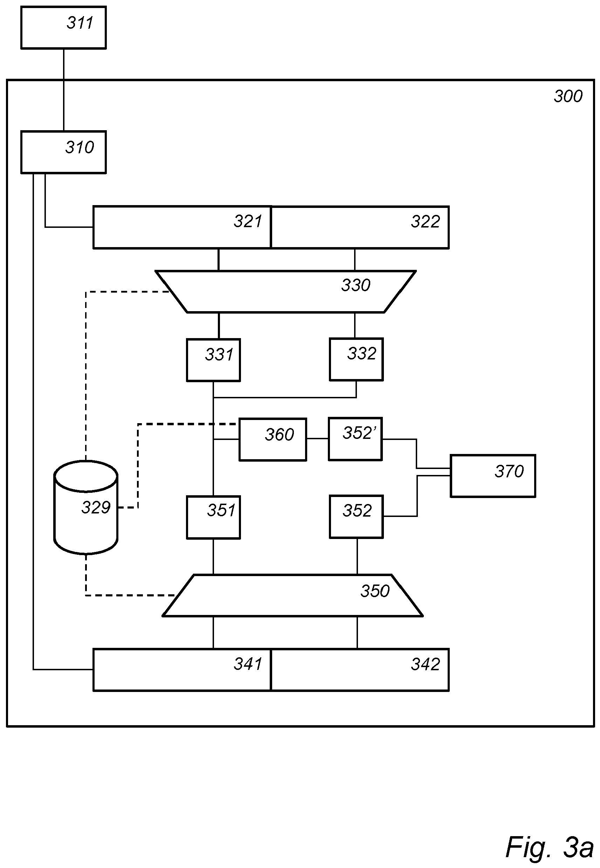

The execution of device 100 and 101 is implemented in a processor circuit, examples of which are shown herein. Figs. 2a-2e, 3a-3b show functional units that may be functional units of the processor circuit. For example, these figures may be used as a blueprint of a possible functional organization of the processor circuit. The processor circuit is not shown separate from the units in these figures. For example, the functional units shown in these figures may be wholly or partially implemented in computer instructions that are stored at device 100 or 101, e.g., in an electronic memory of device 100 or 101, and are executable by a microprocessor of device 100 or 101. In hybrid embodiments, functional units may be implemented partially in hardware, e.g., as coprocessors, e.g., signal coprocessors, and partially in software stored and executed on device 100 and/or 101.

In an embodiment, the fetal heart rate prediction model may comprise a neural network, in particular a deep neural network. For example, the neural network may comprise one or more, two or more, etc., convolutional layers. For example, the neural network may comprise one or more, two or more, etc., fully connected layers. For example, the neural network may comprise one or more, two or more, etc., drop-out layers. For example, in an embodiment, the neural network comprises at least two convolutional layers, at least one dropout layer and at least one fully connected layer. Another type of machine learning classifier is for example, a support vector machine, etc. In an embodiment, drop-out is only used during training, not during operational use when the network is deployed.

Some general aspects of anomaly detection of fetal heart rate (FHR) signals are now described that apply to the specific embodiments that follow.

The FHR is influenced by intrinsic and extrinsic factors. Under normal circumstances, the FHR may be regarded as comprising a baseline FHR, determined by the atrial pacemaker which is modulated by the autonomic nervous system (ANS) via the sympathetic and parasympathetic nervous system, plus some variations around that baseline.

Stimulation of the sympathetic nervous system increases the heart rate and this response tends to be over a long time, causing slow and more sustained accelerations. In contrast, stimulation of the parasympathetic nervous system decreases the heart rate, which typically results in fast decelerations in the FHR.

Low-frequency heart rate variability can be ascribed to the baroreceptor reflex which is under control of the sympathetic and parasympathetic nervous system. In contrast, the high-frequency fluctuations are only under parasympathetic control.

Overall, changes in the FHR can be regarded as being regulated by a combination of sympathetic and parasympathetic activity.

Various embodiments relate to the use of fetal heart rate signals and uterine activity signals. One way to derive these signals, is from CTG recordings. An example of a system to obtain CTG records is the STAN system by Neoventa Medical AB, Molndal, Sweden. To obtain the FHR signal, the fetal electrocardiogram (ECG) may be measured using a scalp electrode directly connected to the fetus. In this ECG signal, the QRS-complexes, which represent the main electrical activity of the heart while it is beating, may be detected. The interval between the QRS-complexes can be used to calculate the FHR.

Fetal heart rate signals used herein may be obtained as a time series of values of the fetal heart rate, e.g. in bpm, for example, at a fixed sample frequency. For example, the frequency may be 4 Hz or similar, e.g., at most or at least 1 Hz, at most or at least 4 Hz, or at most or at least 10 Hz. To obtain the signal at a fixed frequency, a linear interpolation may be used. For example, when using QRS complexes, the FHR may be calculated when a new QRS-complex is detected, so that the FHR obtained from this scalp electrode is not equidistantly sampled. This is an example of a case where linear interpolation may be used to obtain FHR samples at a fixed frequency, e.g., of 4 Hz.

Uterine activity signals may be obtained from a maternal tocogram, by measuring the activity of the uterine muscle with a tocodynamometer, for example, a transducer that is placed on the mother's abdomen. The uterine activity signal, e.g., the TOCO signal, may be sampled at the same fixed frequency as the fetal heart rate signal, e.g., 4 Hz. The signal may be a time series of values representing the uterine contraction, e.g., as a relative measure (e.g., percentage of the maximal contraction) or an absolute measure (e.g., as an amount of pressure e.g. in mmHg or as a number of millimetres or centimetres of contraction).

Fetal heart rate signals and/or uterine activity signals as described herein may optionally be normalized, which may help to speed up the learning process of the model(s) being trained. For example, the fetal heart rate and uterine activity signals may be normalized by first subtracting the mean and then dividing by a given percentile, e.g., the 98th percentile value, to scale the dataset. This normalization may help to avoid focusing the normalization too much on possible outliers in our dataset. The same normalization may be applied to the signals in use.

Fetal heart rate signals and/or uterine activity signals as described herein may also be subject to a signal processing operation. The signal processing may involve a filtering using a zero-phase digital low-pass filter. This filter may help to filter out very abrupt change, primarily to reduce measurement noise. Although this operation may filter away some of the FHR variability, this is not a problem for the applications described herein given that exact high-frequency FHR variability does not need to be predicted.

Both for evaluation purposes and in order to select signals of healthy fetuses to be used for training, a manual labelling by a gynaecologist may be performed. Unhealthy fetuses may have hypoxic ischemic damage. Other indicators of a possibly unhealthy fetus that may be used include congenital abnormalities, meconium-stained amniotic fluid, a low birth weight, pH < 7.05, base deficit > 12 mmol/l, medication that might influence the CTG, maternal or neonatal fever or any missing relevant patient data or CTG recordings, gestation shorter than 36 or longer than 42 weeks. When training the model, signals with some or all of these characteristics may be excluded.

In various embodiments, an anomaly score is determined using a fetal heart rate prediction model, a fetal heart rate encoder model and a uterine activity encoder model. This technique is motivated by the physiology of ANS-mediated FHR variations.

A goal of the models is to discover underlying structures in the FHR signal given variations in UC and preceding FHR data. It is known per se in physiology that variations in the FHR are driven by the sympathetic and parasympathetic nervous branches controlled by the ANS. Uterine contractions provide an external stimulus to the fetal nervous branches. During a UC the ANS could respond with a change in FHR to stabilize the fetal cardiovascular system.

The model structure using a fetal heart rate prediction model, a fetal heart rate encoder model and a uterine activity encoder model, effectively mimics this system. This may result in more accurate and better interpretable predictions of the fetal condition. Uterine activity, e.g., uterine contractions, may be captured in the form e.g. of TOCO measurements, from which the encoder may effectively extract relevant nervous system stimuli. Historic FHR data may also be encoded in order to give the system context about the current fetal condition. These stimuli may then be provided as an input to the fetal heart rate prediction model, which may predict a future (with respect to the first fetal heart rate segment) latent FHR segment. In the physiological analogy, this can be seen as the ANS responding to a given uterine activity, resulting in a certain activity of the sympathetic and parasympathetic nervous systems that control the FHR.

Optionally, a fetal heart rate decoder model may be used to transform this latent prediction into an estimate of the heart rate, similar as the heart adapts its rhythm of contraction based on the inputs it receives from the ANS branches. The predicted fetal heart rate signal may, in use, be output for instance to a clinician, and/or may be used in training to compute a loss, by comparing estimated with measured FHR.

In order to embed the architectural prior from physiology in the network, a two-step training paradigm may be used, in which the encoder and/or decoder models and the prediction model are trained using separate losses. For example, the encoder and/or decoder models may be trained before training the prediction model given the encoder and/or decoder models, or the models may be trained simultaneously using a combined loss that incorporates the respective losses. This may enforce the respective models to have a specific objective in the anomaly detection, resulting in a clearer separation of concerns and thus an architecture that provides more insight on how it operates. Thus, better-explainable predictions may be obtained. It is not needed to use separately trained encoder models in addition to the prediction model, however. For example, the encoder and/or decoder models may be trained end-to-end together with the prediction model. It is also possible to use a prediction model that operates directly on the heart rate and uterine activity segment data, or on signal processed representations of this data without the use of trainable models, for example.

In a training step, an autoencoder such as a VAE may be trained to learn an encoder *genc* and decoder *gdec*, to transform FHR input data into a representation, for example in a continuous latent space. An example is described with respect to Fig. 2a. This autoencoder training may also be used to train a separate encoder and decoder for uterine activity, e.g., TOCO, segments. An example of this is described with respect to Fig. 2b.

Given trained fetal heart rate and uterine activity encoder models, a fetal heart rate prediction model may be used that has the objective to predict new segments of latent FHR data (where the word "prediction" does not mean that the predicted segment necessarily lies in the future; in fact, at the time the prediction is made, the actual measurement that is predicted is often already available and is compared to its prediction). This model may take as an input a previous segment of latent FHR and uterine activity signals, obtained using the encoder from the first step, together with the current uterine activity segment. The model may predict a new latent representation of the current FHR segment. The learned FHR decoder may optionally be used to decode the latent FHR predictions, or the FHR prediction may be further used in the latent representation domain.

**Fig. 2a** schematically shows an example of an embodiment of a device 201 for training a fetal heart rate anomaly detection. **Fig. 2b** schematically shows an example of an embodiment of a device 202 for training a fetal heart rate anomaly detection. Aspects discussed with respect to these figures may be used to implement device 101 of Fig. 1b, and models trained as described with respect to these figures may be applied by device 100 of Fig 1a.

In the example of the figures, segments of FHR and UC data are transformed into a confined latent representation by a variational autoencoder, using variational inference to learn the distribution and providing a lower-dimensional representation of the data.

Fig. 2a shows a uterine activity autoencoder. The uterine activity autoencoder comprises a uterine activity encoder 230 configured to determine an encoding 231 of a uterine activity segment 221. The uterine activity autoencoder further comprises a uterine activity decoder 239 configured to determine a predicted uterine activity segment 221' from encoding 231. The encoder and decoder may comprise trainable parameters accessed from a storage 229, e.g., storage 129 of Fig. 1c. Training 271 may train the autoencoder on training uterine activity data to minimize a difference between the predicted segment 221' and the actual segment 221.

Fig. 2b shows a fetal heart rate autoencoder. The fetal heart rate autoencoder comprises a fetal heart rate encoder 250 configured to determine an encoding 251 of a fetal heart rate segment 241. The fetal heart rate autoencoder further comprises a fetal heart rate decoder 259 configured to determine a predicted fetal heart rate segment 241' from encoding 251. The encoder and decoder may comprise trainable parameters accessed from a storage 229, e.g., storage 129 of Fig. 1c. Training 272 may train the autoencoder on training fetal heart rate data to minimize a difference between the predicted segment 241' and the actual segment 241.

The autoencoders of Fig. 2a and Fig. 2b may be so-called variational autoencoders (VAEs). An encoding 231, 251 may comprise parameters of a probability distribution of a latent representation of the fetal heart rate or uterine activity segment. A VAE seeks to infer the data-generating latent space, capturing characteristics of the data. This can provide better interpretable and disentangled data representations. In addition, VAEAs may have a continuous latent space, allowing efficient sampling which is useful for generative modelling.

Generally, a VAE may model a relationship between two random variables, observed variable **x**, in this case uterine activity segment 221 or fetal heart rate segment 241, and latent variable **z**, in this case a representation 231 of the uterine activity segment 221 or a representation 251 of the fetal heart rate segment 241. The VAE may apply an encoder $q_\phi(\mathbf{z}|\mathbf{x})$, 230, 250 (also referred to as an inference model) that infers the hidden representation 231, 251 of the data 221, 241 to generate the latent variable z. A decoder, $p_\theta(\mathbf{x}|\mathbf{z})$, 239, 259 (also referred to as a generative model) may be configured to approximate the observation 221, 241 given the latent variable 231, 251. The encoder 231, 251 may represent the approximate posterior by parameters of a probability distribution, for example a mean $\boldsymbol{\mu}$ and variance $\sigma$ that are predicted by a model $q_\phi(\mathbf{z}|\mathbf{x}) \sim N(\mu, \sigma^2)$ that takes x, 221, 241 as input. As prior distribution p(z), a normal Gaussian distribution may be used. VAEs 230,239 and 250,259 may be trained by maximizing an evidence lower bound objective (ELBO) comprising a reconstruction component and a regularization component. For example:

$$\log p_\theta(\mathbf{x}) \geq \underbrace{\mathbb{E}_{q_\phi(\mathbf{z}|\mathbf{x})}[\log p_\theta(\mathbf{x}|\mathbf{z})]}_{\text{reconstruction component}} \underbrace{- D_{KL}(q_\phi(\mathbf{z}|\mathbf{x}) \,\|\, p(\mathbf{z}))}_{\text{regularization component}}$$

The reconstruction component may encourage the output 221', 241' of the decoder to match the input 221, 241 of the encoder. A regularization component may be included to improve generalizability of the model. In the above example, $D_{KL}(q_\phi(\mathbf{z}|\mathbf{x}) \,\|\, p(z)$ is the Kullback-Leibler divergence between the prior and posterior distributions. Training operations 271, 272 may optimize for the ELBO using a technique known as the "reparameterization trick".

An encoder model 230, 250 may comprise one or more convolutional layers. A convolutional layer may be used to create feature maps of segments of input data. A convolutional layer may be followed by a max-pooling layer, which shrinks the size of the segment representation. Combinations of these two layers may be repeated and concatenated behind each other, shrinking the size of the segment representation further with every concatenation. As final layers of the model, one or more dense layers may be used. The dense layers may be used to map the rich feature maps determined by the convolutional layers into an output 231, 251 comprising parameters of a probability distribution, for example, a predicted mean $\boldsymbol{\mu} \in \mathbb{R}^N$ and variance $\boldsymbol{\sigma} \in \mathbb{R}^N$ vectors of a Gaussian distribution.

The decoding 239, 259 of the representations 231, 251 may involve a sampling operation, where samples are taken from the probability distributions defined by representations 231, 251. Such sampling may be incompatible with training by backpropagation of gradient due to it being non-deterministic. To account for this, training 271, 272 may apply a reparameterization method as described for example in D. Kingma and M. Welling, "Auto-Encoding Variational Bayes" (available at https://arxiv.org/abs/1312.6114 and incorporated herein by reference). According to this method, for the purpose of the training, the random sampling may be dealt with as an input to the encoder model 239, 259 instead of a process that happens within the decoder or encoder model 239, 259. Such a reparameterization may be implemented as:

$$z = \boldsymbol{\mu} + \boldsymbol{\sigma} \odot \epsilon$$

where $\epsilon \sim N(0, I)$.

The decoder may reconstruct input segment 221, 241 by sampling from the latent space distributions 231, 251 and feeding these samples through a series of convolutional layers. A convolutional layer may generate features dependent on z, with z the samples taken from the latent space. After a convolutional layer, an upsampling layer may be applied which may repeat a temporal step along the time axis to increase the dimensionality of the representation. The series of these two layers may be repeated until a dimensionality that matches the input segment of the encoder is obtained. Many variations are possible, e.g. normalizing flows may be incorporated in the reconstruction network.

The models may be trained by minimizing a loss function. This loss function may be derived from the above-mentioned ELBO including the reconstruction component and a regularization component.

Gaussian priors may be used, in which case the reconstruction component may be equal to a mean squared error loss, reflecting the difference between the input segment 221, 241 and the reconstructed segment 221', 241', and may be defined as:

$$\mathcal{L}_{\mathrm{mse}}(\mathbf{x}, \hat{\mathbf{x}}) = \frac{1}{n}\sum_{i=0}^{n} (\mathbf{x}_i - \hat{\mathbf{x}}_i)^2$$

where n is the number of training samples, x the input segment 221, 241 to the VAE and $\hat{\mathbf{x}}$ the output reconstructed segments 221', 241' of the VAE.

The second component is a regularizer for the latent space, which can be a Kullback-Leibler divergence, which is a measure of how close the encoder output distribution is to a predefined target distribution, e.g., a Gaussian. For example, the KL divergence may be computed as:

$$\mathcal{L}_{\mathrm{KL}}(\boldsymbol{\mu}, \boldsymbol{\sigma}) = -\frac{1}{2}\sum_{j=0}^{N-1} (1 - \mu_j^2 - \sigma_j^2 + log(\sigma_j + \rho)^2)$$

where a constant $\rho$, e.g., $\rho = 1 \cdot 10^{-8}$, may be added for numerical stability. This regularizer component may help to obtain more diverse latent representations z 231, 251 forming a more meaningful representation of the input segments 221, 241. Interestingly, it is also possible to adapt latent representations and feed these samples to the decoder 239, 259, generating a new segment of data. This may be used to generate new segments of predicted FHR data using previous latent variable vectors. These two losses above may be used to obtain the following total loss that can be optimized in training 271, 272:

$$\mathcal{L}_{\mathrm{VAE}} = \alpha_1 \mathcal{L}_{\mathrm{mse}} + \alpha_2 \mathcal{L}_{\mathrm{KL}}.$$

For the regularizer, a cyclical annealing schedule may be used to reduce the vanishing of this term and to allow the network to first learn more meaningful latent codes during training. It was found that $\alpha_1 = 1$ and a final $\alpha_2 = 0.001$ yields a good trade-off between reconstruction loss and regularization loss in the network. The weights of the model may be initialized randomly from a uniform distribution. Training 271,272 may be performed using stochastic gradient descent.

Good results were obtained with the Adam optimizer with a learning rate of 0.01. It was found that a VAE can make high-quality reconstructions of input segments of data with only small deviations from the reference. The encoder and decoder can capture relevant features of the FHR data and the uterine activity data in the latent space representation, as evidenced by the fact that the network can consistently generate high-quality predictions. A Gaussian-like shape of the distribution of the latent space was obtained when using the Kullback-Leibler divergence loss discussed above, which is beneficial to allow continuous sampling from this latent space.

It is not needed to train the encoder models 230, 250 together with corresponding decoder models. As an alternative, for example, the encoder models, 230, 250 may be trained using Contrastive Predictive Coding or similar techniques. Such training may not result in a corresponding decoder model 239, 259. As also discussed elsewhere, prediction models as described herein can be trained and used also in such cases.

**Fig. 2c** schematically shows an example of an embodiment of a device for training a fetal heart rate anomaly detection. Aspects discussed with respect to this figure may be used to implement device 101 of Fig. 1b, and models trained as described with respect to this figure may be applied by device 100 of Fig 1a.

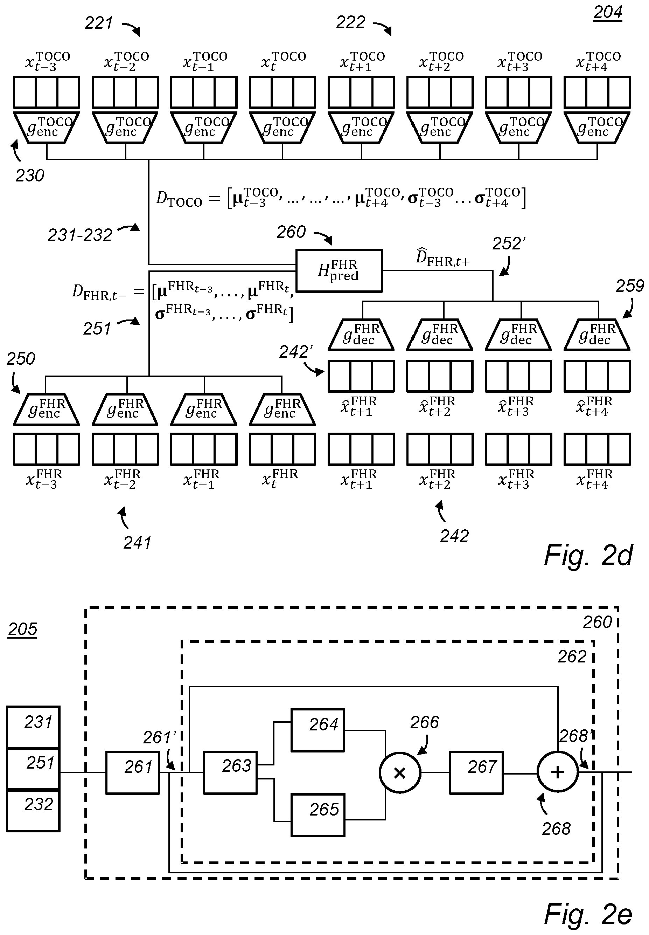

Shown in the figure is a fetal heart rate prediction model 260 configured to determine a predicted representation 252' of a second fetal heart rate segment from a representation 251 of a first fetal heart rate segment 241, a representation 231 of a first uterine activity segment 221, and a representation 232 of the second uterine activity segment 222. In this example, the representations are determined by a fetal heart rate encoder model 250 and a uterine activity encoder model, 230, respectively, that have been trained separately before training the prediction model 260, as discussed with respect to Fig. 2a-2b. It is also possible to train the fetal heart rate prediction model 260 simultaneously with the encoder and/or decoder models of Fig. 2a-2b, e.g., by using a combined loss that incorporates the losses of the respective models. Another alternative is to use a fetal heart rate prediction model 260 that takes the segments 221,222,241 directly as input, or that is trained end-to-end with the encoder model(s) 230, 250 and decoder model 259 (if using) using a single loss.

The prediction model 260 may be configured to determine a predicted representation 252' of the same shape as an output of the fetal heart rate prediction model 250, e.g., in the form of distribution parameters for the second FHR segment. Generally, various model architectures can be used for the prediction model, e.g., the model 260 can be a convolutional neural network. Detailed examples of model architectures are provided herein. Although not shown here, the prediction model 260 can have other inputs in addition to representations 231, 232, 251. In particular, the model may be provided with additional information about the mother and child, for example, one or more clinical parameters that can influence the FHR, such as a gestational age and/or information about medication. This can allow the model to learn new dependencies whereby the predictions of the model may be improved.

The prediction model may be trained in a training operation 273. For the training, a two-component loss function may be used similar to the loss function of Fig. 2a-2b, comprising a prediction component and a regularizer component.

The prediction component of the loss function may use a fetal heart rate decoder model 259 corresponding to the encoder model 250, as discussed with respect to Fig. 2b for example. The training 273 may be performed by applying the fetal heart rate prediction model 260 to obtain a predicted encoding 252' of the second fetal heart rate segment; applying the fetal heart rate decoder model 259 to the predicted encoding 252' to obtain a predicted fetal heart rate segment 242'; and comparing the predicted fetal heart rate segment 242' to the corresponding fetal heart rate segment 242 of the training dataset.

The comparison may be computed for example as a mean squared error reflecting the difference between the predicted segment 242' and actual segment 242, e.g.:

$$\mathcal{L}_{\mathrm{mse,d}}(\mathbf{x}, \hat{\mathbf{x}}) = \frac{1}{n} \sum_{i=0}^{n} (\mathbf{x}_i - \hat{\mathbf{x}}_i)^2 \beta_i, (7)$$

where $\beta_i$ is an optional linear decaying factor applied to the loss, reducing the penalty on predictions that are further forward in time.

The second component may be a regularizer for the predicted latent space, for example, a, Kullback-Leibler divergence The prediction component and the regularizer component of the loss may be combined to obtain an overall loss for prediction model 260, e.g.:

$$\mathcal{L}_{\mathrm{pred}} = \alpha_1 \mathcal{L}_{\mathrm{mse}} + \alpha_2 \mathcal{L}_{\mathrm{KL}}.$$

Values $\alpha_1 = 1$ and $\alpha_2 = 0.005$ were found to provide a good trade-off between reconstruction-loss and KL-loss of the network.

In the above example, the reconstruction loss is computed in the domain of the fetal heart rate segments. As an alternative, a reconstruction loss may be computed by comparing the representation 252' output by the prediction model 260 to a representation of the fetal heart rate segment 242 obtained by applying the fetal heart rate encoder 250. This alternative can be used, for example, if no decoder model is available.

Fig. 2d schematically shows an example of an embodiment of a device 204 for training a fetal heart rate anomaly detection. This example is based on, and can be used in implementations of, the examples of Fig. 2a-2c.

This example demonstrates the use of segmentwise encodings. As in Fig. 2a-2c, a uterine activity encoder model 230 is shown that is applied to first uterine activity segment 221 and a second uterine activity segment 222. In this figure, a uterine activity segment 221, 222 comprises multiple subsegments $x_i^{\mathrm{TOCO}}$. As an illustrative example, four subsegments are shown. A subsegment itself may comprise multiple samples of a uterine activity value, e.g., of an amount of contraction. As an illustrative example, four samples per subsegment are shown in the figure. The actual number of samples per segment is usually higher, e.g., at most or at least 32 samples, at most or at least 64 samples or at most or at least 128 samples.

As shown in the figure, the uterine activity encoder model 230 may be applied subsegment-per-subsegment to obtain respective outputs for the subsegments. The respective outputs may be concatenated to obtain representations 231, 232 of the uterine activity segments. For example, the uterine activity encoder model 230 may apply one or more convolutional layers and/or one or more dense layers to a respective subsegment, as discussed with respect to Fig. 2a-2b. The uterine activity encoder model may be trained as an autoencoder together with a uterine activity decoder model that also operates subsegment-per-subsegment, e.g., by applying one or more convolutional layers as discussed with respect to Fig. 2a-2b.

Similarly, in this example, also the fetal heart rate encoder model 250 operates in a subsegment-per-subsegment fashion. The first and second fetal heart rate segments 241, 242 may each comprise respective subsegments $x_i^{\mathrm{FHR}}$.

A subsegment may itself comprise a number of samples, e.g., of a fetal heart rate value. The number of samples per subsegment may be at most or at least 32 samples, at most or at least 64 samples or at most or at least 128 samples, for examples. The number of samples can be the same as the number of samples per uterine activity subsegment, but this is not strictly needed.

Fetal heart rate encoder model 250 may be applied by determining a subsegment-by-subsegment encoding of the fetal heart rate segment, e.g., to obtain representation 251 of the first fetal heart rate segment 241. The fetal heart rate encoder model may have a similar model architecture as discussed for uterine activity encoder model 230. The figure also shows a fetal heart rate decoder model 259 that can for example be trained together with the fetal heart rate encoder model 250 as an autoencoder. Also this fetal heart rate decoder model may operate in a subsegment-per-subsegment fashion. In this example, it is applied to predicted representation 252' of the second fetal heart rate segment 242, in a subsegment-per-subsegment fashion, to obtain a predicted fetal heart rate segment 242'. The fetal heart rate decoder model 259 may have a model architecture as discussed for the uterine activity decoder model.

The representations 231, 232, 251 output by the encoder models 230, 250 are typically smaller than the segments 221, 222, 241 to which they are applied, for example, by a factor 4 or greater, by a factor 8 or greater, of by a factor 16 or greater. For example, a subsegment comprising 64 samples may be represented by an 8-D latent representation comprising 8 means and 8 variances of a Gaussian distribution. Although the number of samples per segment is typically fixed, the encoders and decoders 230, 250, 259 may be applied to segments comprising an arbitrary number of subsegments.

The figure also shows the fetal heart rate prediction model 260 being applied to the representation 251 of the first fetal heart rate segment, the representation 231 of the first uterine activity segment, and the representation 232 of the second uterine activity segment, to obtain the predicted representation 252' of the second fetal heart rate segment. The fetal heart rate prediction model is typically configured to operate on representations 231, 232, 251 of a fixed length, e.g., representing a fixed number of subsegments. For example, the prediction model 260 may operate on segments 231, 232, 251 comprising 16 subsegments, and may produce a prediction 252' comprising 4 subsegments. The number of predicted subsegments is typically smaller than the number of input segments, e.g., by a factor at least 4. The number of input segments can be at most or at least 4, at most or at least 16, or at most or at least 64, for example.

As shown in the figure, fetal heart rate decoder model 259 may be applied to the predicted representation of the second fetal heart rate time segment 252' to obtain a prediction 242' of the second fetal heart rate time segment, for example for comparison to a fetal heart rate time segment 242 that was actually measured. A consequence of applying the decoder model 259 segment-per-segment, is that the decoder 259 can produce FHR segments that show discontinuities at the points where two segments meet. To reduce these discontinuities, a subsegment transition smoothening may be applied to the segment-by-segment outputs of the decoder 259 to obtain the predicted fetal heart rate segment 242'. This can be done during training and/or in use. For example, the subsegment transition smoothening may be implemented as a trainable convolutional layer, such as a dilated convolutional layer.

**Fig. 2e** schematically shows an example of an embodiment of a device 205 for training a fetal heart rate anomaly detection. This example is based on, and can be used in, the examples of Fig. 2c and Fig. 2d.

This example shows fetal heart rate prediction model 260 being applied to a representation 251 of the first fetal heart rate segment, a representation 231 of the first uterine activity segment, and a representation 232 of the second uterine activity segment, to obtain a predicted representation of the second fetal heart rate segment. For example, the representations can be segmentwise encodings as discussed with respect to Fig. 2d.

In this example, the fetal heart rate prediction model 260 is applied by applying a causal convolutional layer 261, followed by one or more residual blocks 262. The causal convolutional layer 261 is used to embed the sequential temporal structure of the time segments 231,251,232 in the model. The residual blocks progressively construct the prediction. For example, the number of residual blocks may be at most or at least two, at most or at least five, or at most or at least ten.

The causal convolutional layer and residual blocks may form a deep neural network. An architecture of this type is known from A. van den Oord et al., "WaveNet: A Generative Model for Raw Audio" (available at https://arxiv.org/abs/1609.03499 and incorporated herein by reference) and can be used as a basis of prediction model 260. Interestingly, models of this type may be fully probabilistic, with a predictive distribution that is based on all previous segments of input data. Moreover, such models can be efficiently trained on long sequences of data, capturing long and short-term dependencies in the data, resulting in good performance while also capturing specific characteristics. WaveNet itself is trained on speech segments, but interestingly, the inventors realized that this model and variations of it can be applied to time segments of fetal heart rate and uterine activity signals as well.

Generally, a residual block 262 may be configured to determine a final block output 268' from a block input 261' by applying one or more layers to the block input to obtain a block output, and combining the block output with the block input 261' according to a skip connection 268. For example, the fetal heart rate prediction model 260 may be an artificial neural network comprising residual block(s) 262, also known as a residual neural network.

A particular example of an architecture of a residual block 262 is shown in the figure. The residual block 262 comprises a dilated convolutional layer 263 that is used to increase the size of the receptive field of the model, while preserving the resolution throughout the model. The dilated convolutional layer is followed by a double-gated activation 266. The double-gated activation may comprise an activation filter 264 for dilated convolutional layer 263, for example, a hyperbolic tangent (tanh) branch. The double-gated activation may also comprise a branch 265 that is used to decide which past data is relevant to keep, e.g., a sigmoid branch. As shown in the figure, a 1x1 convolution 267 may be applied to the result of the double-gated activation. The shown residual block also comprises the skip connection 268. For example, the skip connection may be implemented as described in K. He et al., "Deep residual learning for image recognition", Proceedings of the IEEE Computer Society Conference on Computer Vision and Pattern Recognition, 2016 (incorporated herein by reference). The skip connection 268 provides an alternative path for the gradient to flow and increases the model convergence rate. The figure shows a residual skip connection, which is the preferred option, although it is also possible to use parameterized skip connections instead or in addition.

**Fig. 3a** schematically shows an example of an embodiment of a device 300 for processing a fetal heart rate signal, for example, based on device 100 of Fig. 1a. In particular, device 300 may be for performing an anomaly detection on the fetal heart rate signal. The device of this example uses a trained uterine activity encoder model 330, a trained fetal heart rate encoder model 350, and a trained fetal heart rate prediction model 360. The models 330, 350, 360 may have been trained as described herein, e.g., according to the techniques of Fig. 2a-2e. In particular, the models may be parameterized by model data accessed from a storage 329 and trained as described herein.

The figure shows a sensor interface 310 for accessing a fetal heart rate signal of a fetus and a corresponding uterine activity signal, e.g., based on sensor interface 110 of Fig. 1a. The figure illustrates the use of multiple electrodes 311 arranged on the abdomen of a pregnant woman, but other ways of obtaining the signals are possible as well. Via the sensor interface 310, a first fetal heart rate segment 341 and a subsequent (e.g., directly following in time) second fetal heart rate segment 342 of the fetal heart rate signal may be obtained. Further, via the sensor interface 310, a first uterine activity segment 321 and a second uterine activity segment 322 of the uterine activity signal may be obtained, corresponding temporally to the first and second fetal heart rate segments 341 and 342, respectively. For example, the segments may comprise samples and/or may be subdivided into subsegments, as also described elsewhere.

As shown in the figure, the uterine activity encoder model 330 may be applied to the first and second uterine activity segments 321, 322 to obtain inferred encodings 331, 332 representing the first and second uterine activity segments. The figure also shows the fetal heart rate encoder model 350 being applied to the first and second fetal heart rate segments 341, 342 to obtain inferred encodings 351, 352 representing the first and second fetal heart rate segments.

The fetal heart rate prediction model 360 may be applied to the inferred encoding 351 of the first fetal heart rate segment, the inferred encoding 331 of the first uterine activity segment, and the inferred encoding 332 of the second uterine activity segment to obtain a predicted encoding 352' representing the second fetal heart rate segment.

The predicted encoding 352' may be used for anomaly detection by performing a comparison 370 of the predicted representation 352' of the second fetal heart rate segment to the second fetal heart rate segment 342 that was actually measured, based on which an anomaly score may be determined. Effectively, this anomaly detection creates a virtual image of what the FHR would look like, given the CTG thus far and the presence or absence of UC in the segment to come, and compares this virtual image with the actual FHR response to UC in this segment. Given this comparison, it may be assessed whether the fetus is responding in a way that is expected from a healthy fetus and thus whether the fetus is expected to be in good health.

The comparison 370 is preferably made in terms of representations 352, 352', and not in terms of the fetal heart rate segments themselves. Compared to Fig. 2c-2d, application of decoder model 259 (including the sampling from the latent distribution), may be omitted. Comparing representations may provide better results than comparing segments, since the representations essentially provide a compressed representation in which different aspects of the signal are separated out and can thus be easily compared latent-per-latent.

The latent FHR distribution 352' may comprise parameters of a probability distribution of a latent representation of the fetal heart rate segment, e.g., represented by parameters of $N$ Gaussians, where N is the dimensionality of the latent space. For example, the representation 352' may comprise latent representations for respective subsegments. This representation 352' may be compared to a corresponding representation 352 of the second fetal heart rate segment obtained by applying fetal heart rate encoder model 350 to second fetal heart rate segment 342.

In particular, the comparison 370 may be performed by computing a divergence between the parameters of the probability distributions defined by the predicted and inferred encodings 352, 352'. In particular, the predicted versus calculated distributions can be computed by computing a KL-divergence between the two distribution vectors, e.g., for respective encoded dimensions and/or in a subsegment-per-subsegment fashion. For example, the divergence for a particular latent of a particular subsegment, represented as a Gaussian distribution, may be computed as:

$$KL[p,q] = log\left(\frac{\sigma_2}{\sigma_1}\right) + \frac{\sigma_1^2 + (\mu_1 - \mu_2)^2}{2\sigma_2^2} - \frac{1}{2},$$

where p(x) = $N(\mu_1, \sigma_1)$ is the distribution of the encoded measured segment 352 and q(x) = $N(\mu_2, \sigma_2)$ is the distribution of the encoded predicted segment 352'.

Comparison 370 may combine the divergences of respective latent variables into an overall anomaly score. This can be done in various ways, e.g., as an average, as a maximum, or by detecting per dimension in the latent space whether the value of the divergence exceeds a given threshold. It was found in practice that, if an anomaly is present in the prediction 352', it tends to be present throughout the latent variables of the prediction, so that many different ways of combining the divergences can work. The anomaly score may be a continuous value, a prediction of the fetal condition, being either healthy or anomalous, a 1-5 or 1-100 score, or another discrete value, etc. The anomaly score can also be per subsegment or for the overall segment predicted by the model 360.

Since the FHR predictor model 360 is typically only trained on healthy fetuses, essentially modelling the response of a healthy fetus to UC, a large divergence may indicate a discrepancy between the two distributions and therefore possibly an anomalous event. Interestingly, data of unhealthy fetuses, i.e., anomalies, is not needed.

It is noted that, when using a divergence to perform the comparison 370 as above, this comparison itself may not need to be trained. Instead it is based on trained models for predicting the responses to uterine activity of healthy fetuses. In other embodiments, further training or other customization of the comparison may be used to obtain a more meaningful comparison, however. In particular, since encoder models 330, 350 typically provide disentangled latent variables, respective dimensions of the predicted latent space 352' may be expected to refer to respective semantically meaningful concepts, or may be more informative than others for other reasons. By weighing informative latent variables more heavily, e.g., manually or automatically, the comparison 370 may be improved.

Instead of performing comparison 370 in terms of encodings 352, 352', it is also possible to directly compare the predicted and measured fetal heart rate segments. To this end, a fetal heart rate decoder model may be applied to predicted encoding 352' and the resulting predicted fetal heart rate segment may be compared to the measured fetal heart rate segment 342, e.g., similar to the comparison performed between segments 242, 242' by training 273 of Fig. 2c.

It is also possible to apply a fetal heart rate decoder model to the predicted encoding 352' to obtain a predicted second fetal heart rate segment, and to output the predicted and/or measured second fetal heart rate segments to a user. The output can be visualized in combination with the determined anomaly scores, for example. For example, a CTG recording may be shown with the background colour indicating the divergence between a predicted FHR segment and the measured FHR segment and thus indicating possible anomalous regions.

Also the predicted latent representation 352' may be provided as an output. For example, it may be possible to link certain fetal behaviour to the activity of a specific latent, e.g., specific latents may represent parasympathetic or sympathetic activity of the fetus, and may thus be used as outputs informative of these aspects.

The comparison 370 may be configured to determine anomaly scores at multiple points in time and to aggregate the anomaly scores into an overall anomaly score. Such an overall anomaly score may be helpful to determine a moment of possible intervention.

For example, the overall anomaly score may be determined performing continuous predictions of fetal health based on the determined anomaly scores according to a trainable model. The model may be trained by verifying these continuous predictions against measured outcomes.

For example, pH measurement may be used as a proxy for the outcome. In practice, gynaecologists often use pH measurements as a tool to assess the health of a fetus. The pH is measured by fetal scalp blood sampling (FBS), for example. In case of deteriorating fetal condition, the pH may progressively decrease as the fetus will resort to anaerobic metabolism when deprived of oxygen. The measurement of pH has several disadvantages: the method to obtain the blood is invasive, non-continuous, and technically uneasy, leading to a high rate of failed blood samplings and a high variability in measurements due to the site of scalp puncture and other agents that alter the pH.

By using an overall anomaly score, or in other words, an overall score of fetal health based on anomaly scores determined as herein, these advantages of pH measurements may be overcome. For example, the overall anomaly score may be configured to predict fetal pH and may be determined by a model trained based on known pH measurements.

For example, the model for predicting the overall anomaly score may be trained based on pH measurements directly after birth by assumed a normal start pH at the beginning of the recording, e.g., be equal to 7.35. The pH may be predicted overtime by looking at the severity and length of an anomalous episode and therefore predicting the impact on the fetus. One possible approach is a linear interpolation to assign drops in pH to respective anomaly scores. Recovery of the pH in case there are no anomalies can be taken into account.

The model for predicting the overall anomaly score can optionally be a probabilistic model. This makes it possible to not only get an estimate of the overall score, e.g., the predicted pH, but also to get an indication of the uncertainty of this estimate. Whenever this uncertainty becomes too high the gynaecologist could decide to take another FBS reducing the uncertainty of the model, for example. The probabilistic model may be updated at a time step with new CTG data, together with FBS whenever measured to infer the actual fetal pH and reset possibly accumulated uncertainties of the CTG-based pH estimate. Instead of pH, also other measures may be used that indicate fetal well-being, such as hypoxic damage.

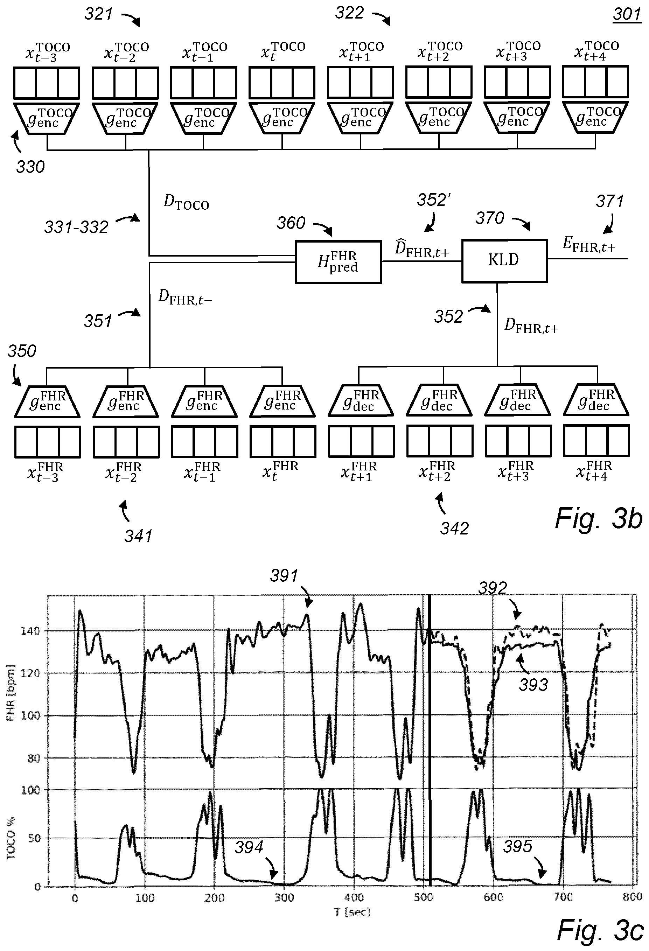

**Fig. 3b** schematically shows an example of an embodiment of a device for processing a fetal heart rate signal. This example is based on the example of Fig. 2d and shows how subsegment-by-subsegment encoders of the fetal heart rate and uterine activity segments may be used.

The figure shows uterine activity segments 321, 322 that comprise multiple subsegments. Uterine activity encoder model 330 may determine subsegment-by-subsegment encodings 331, 332 of the uterine activity segment 321, 322. Also the fetal heart rate segments 341, 342 may comprise multiple subsegments, with the fetal heart rate encoder model 350 being configured to determine subsegment-by-subsegment encodings 351, 352 of fetal heart rate segments 341, 342. Fetal heart rate prediction model 360 may further determine a subsegment-by-subsegment prediction 352' of the encoding of the second fetal heart rate segment 352. The predicted and actual encodings 352, 352' may be compared, 370, in a subsegment-by-subsegment way to determine an anomaly score 371 for the second fetal heart rate segment 342, e.g., as a divergence between probability distributions represented by the encodings.

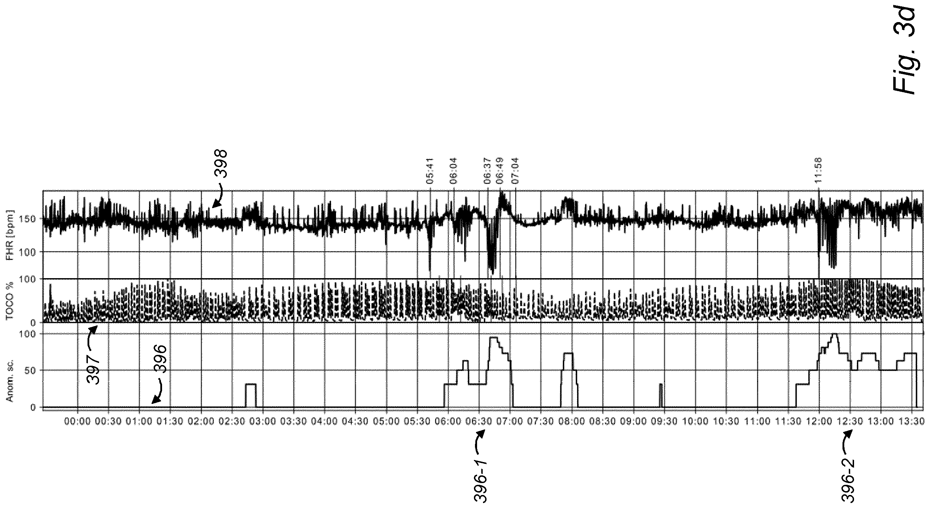

**Fig. 3c** shows an example of a fetal heart rate segment predicted according to the techniques described herein. **Fig. 3d** shows an example of an anomaly detection of a fetal heart rate segment according to the techniques described herein.

Fig. 3c shows a second FHR segment 393 predicted by using a fetal heart rate prediction model to predict a representation of a second fetal heart rate segment, and applying a fetal heart rate decoder model to the representation. The figure also shows measured first FHR segment 391 and second FHR segment 392 in bpm, and corresponding uterine activity signals 394-395 in % of maximal contraction. In this example, an FHR segment 393 of 256 seconds is predicted, based on 512 seconds of preceding FHR and TOCO samples 391, 394 and 256 seconds of current TOCO samples 395.

As the figure shows, the provided techniques are able to accurately predict new segments of FHR data. Over a validation set, a mean absolute error (MAE) of 7.12 bpm for the FHR prediction was found. It was also observed that the model is able to model correlations between UC and predicted FHR segments.

Fig. 3d shows a CTG recording comprising a fetal heart rate signal 398 in bpm and a uterine activity signal 397 in %, to which the described anomaly detection was applied. The figure shows anomaly scores 396 on a 1-100 scale for respective segments of the fetal heart rate signal, determined as described herein. Quality of the determined anomaly scores was verified on a part of the dataset by letting an experienced gynaecologist assess the CTG recordings to indicate possible anomalous episodes. This gynaecologist had full access to the CTG recording, the anamnesis, but also the perinatal outcome, making it possible to reverse engineer possible anomalous events based on the knowledge of a poor outcome. Generally, a high agreement was found between the anomaly scores and the assessment by the gynaecologist. In particular, in the example of Fig. 3d, the gynaecologist made the following assessment:

| Timestamp | Comment |
|---|---|
| 23:24 | Start registration |
| till 05:41 | (more or less) flawless |
| 05:41 | 3 minor incidents, but full recovery |
| 06:04 | start challenge; late deceleration with full recovery |
| 06:37 - 06:49 | late deceleration with incomplete recovery |
| 06:49 - 07:04 | recovery phase |
| 07:04 - 11:58 | normal |
| 11:58 | start challenge, late deceleration with incomplete recovery |
| 13:35 | birth (pH = 7.23) |

In particular, as can be observed from the assessment, the gynaecologist identified the anomalous regions 396-1 and 396-2 in which a high anomaly score persisted over a relatively long time.

In the various embodiments of the fetal heart rate signal processing devices, e.g., device 100, 300, 301, and the training devices, e.g., device 101, 201-205, communication interfaces may be selected from various alternatives. For example, communication interface may be a network interface to a local or wide area network, e.g., the Internet, a storage interface to an internal or external data storage, a keyboard, an application interface (API), etc.

The signal processing and training devices may have a user interface, which may include well-known elements such as one or more buttons, a keyboard, display, touch screen, etc. The user interface may be arranged for accommodating user interaction, e.g., for performing an anomaly detection or for performing a training.

The storages, e.g., storage 129, 229, 329 may be implemented as an electronic memory, say a flash memory, or magnetic memory, say hard disk or the like. A storage may comprise multiple discrete memories together making up the storage.

Typically, a signal processing and training device, e.g., devices 100, 101 each comprise a microprocessor which executes appropriate software stored at the device; for example, that software may have been downloaded and/or stored in a corresponding memory, e.g., a volatile memory such as RAM or a non-volatile memory such as Flash. Alternatively, the devices may, in whole or in part, be implemented in programmable logic, e.g., as field-programmable gate array (FPGA). The classification and training devices may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc.

In an embodiment, a signal processing device comprises a fetal heart rate prediction circuit, and an anomaly scoring circuit. The signal processing device may also comprise fetal heart rate encoding and/or decoding circuits, and/or uterine activity encoding and/or decoding circuits. In an embodiment, a training device comprises a fetal heart rate prediction training circuit. The training device may also comprise fetal heart rate encoding and/or decoding and/or autoencoder training circuits, and/or uterine activity encoding and/or decoding and/or autoencoder training circuits. The circuits implement the corresponding functionality described herein. The circuits may be a processor circuit and storage circuit, the processor circuit executing instructions represented electronically in the storage circuits.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only. The circuits may also be, FPGA, ASIC or the like.

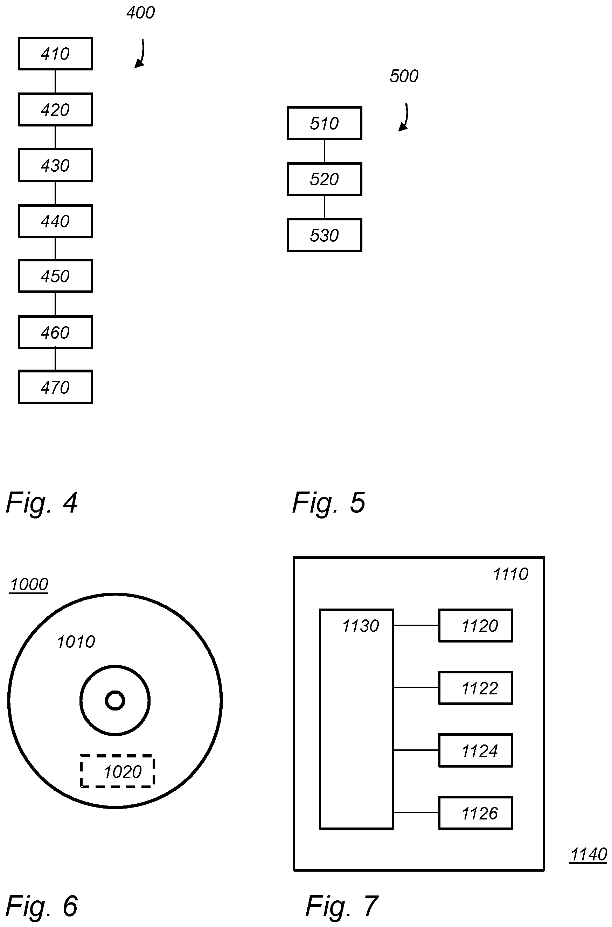

**Fig. 4** schematically shows an example of an embodiment of a computer-implemented method 400 of processing a fetal heart rate signal.

The method 400 may comprise accessing 410 a fetal heart rate signal of a fetus and a corresponding uterine activity signal.

The method 400 may comprise accessing 420 model data representing a fetal heart rate prediction model configured to, based on an encoding of a first fetal heart rate segment, an encoding of a first uterine activity segment, and an encoding of a second uterine activity segment, predict an encoding of a second fetal heart rate segment.

The method 400 may comprise obtaining 430 a first fetal heart rate segment and a subsequent second fetal heart rate segment of the fetal heart rate signal.

The method 400 may comprise obtaining 440 a first uterine activity segment and a second uterine activity segment of the uterine activity signal, corresponding respectively to the first and second fetal heart rate segments.

The method 400 may comprise applying 450 the fetal heart rate prediction model to a representation of the first fetal heart rate segment, a representation of the first uterine activity segment, and a representation of the second uterine activity segment to obtain a predicted representation of the second fetal heart rate segment.

The method 400 may comprise determining 460 an anomaly score indicative of fetal health by comparing the predicted representation of the second fetal heart rate segment to the obtained second fetal heart rate segment.

The method 400 may comprise outputting 470 the anomaly score.

**Fig. 5** schematically shows an example of an embodiment of a computer-implemented method 500 of training a fetal heart rate anomaly detection.

The method 500 may comprise accessing 510 a training dataset comprising multiple fetal heart rate signals of fetuses and corresponding uterine activity signals;

The method 500 may comprise, using the fetal heart rate signals and corresponding uterine activity signals of the training dataset, training 520 a fetal heart rate prediction model to, based on a representation of a first fetal heart rate segment, a representation of a corresponding first uterine activity segment, and a representation of a subsequent second uterine activity segment, predict a representation of a second fetal heart rate segment.

The method 500 may comprise outputting 530 the trained fetal heart rate prediction model.

Many different ways of executing the methods are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started. The methods may be combined, e.g., method 400 may be used to perform a fetal heart rate anomaly detection based on the training of method 500.

Embodiments of the method may be executed using software, which comprises instructions for causing a processor system to perform method 400 and/or 500. Software may only include those steps taken by a particular sub-entity of the system. The software may be stored in a suitable storage medium, such as a hard disk, a memory device, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the invention also extends to computer programs, particularly computer programs on or in a carrier, adapted for putting the invention into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth.

**Fig. 6** shows a computer readable medium 1000 having a writable part 1010. The writable part may comprise a computer program 1020, the computer program 1020 comprising instructions for causing a processor system to perform a signal processing and/or training method, according to an embodiment. Instead or in addition, the writable part may comprise model data 1020 representing a uterine activity encoder model, a fetal heart rate encoder model, and/or a fetal heart rate prediction model. The models may be for use according to a method described herein, and/or trained according to a method described herein. The computer program and/or model data 1020 may be embodied on the computer readable medium 1000 as physical marks or by means of magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said signal processing and/or training method.

**Fig. 7** shows in a schematic representation of a processor system 1140 according to an embodiment. The processor system comprises one or more integrated circuits 1110. The architecture of the one or more integrated circuits 1110 is schematically shown in Figure 12b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively. For example, communication element 1126 may be arranged to receive ECG signals from multiple electrodes, either directly or indirectly, or to receive other data, e.g., training data, trained parameters and the like.

For example, in an embodiment, processor system 1140, e.g., the signal processing and/or training device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of" when preceding a list of elements represent a selection of all or of any subset of elements from the list. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

In the claims, references in parentheses refer to reference signs in drawings of exemplifying embodiments or to formulas of embodiments, thus increasing the intelligibility of the claim. These references shall not be construed as limiting the claim.

**Claims**

1.  A device (100, 300-301) for processing a fetal heart rate signal, comprising:

    - a sensor interface (110, 310) for accessing a fetal heart rate signal of a fetus and a corresponding uterine activity signal;
    - a data interface (120) for accessing model data representing a fetal heart rate prediction model configured to, based on a representation of a first fetal heart rate segment, a representation of a first uterine activity segment, and a representation of a second uterine activity segment, predict a representation of a second fetal heart rate segment;
    - a processor subsystem (130) configured to:

      - obtain, via the sensor interface, a first fetal heart rate segment and a subsequent second fetal heart rate segment of the fetal heart rate signal;
      - obtain, via the sensor interface, a first uterine activity segment and a second uterine activity segment of the uterine activity signal, corresponding respectively to the first and second fetal heart rate segments;
      - apply the fetal heart rate prediction model to a representation of the first fetal heart rate segment, a representation of the first uterine activity segment, and a representation of the second uterine activity segment to obtain a predicted representation of the second fetal heart rate segment;
      - determine an anomaly score indicative of fetal health by comparing the predicted representation of the second fetal heart rate segment to the obtained second fetal heart rate segment, and output the anomaly score.

2.  The device (100, 300-301) of claim 1, wherein the anomaly score is indicative of whether the fetus shows an abnormal reaction of the fetal heart rate to the uterine activity, thereby indicating fetal health.

3.  The device (100, 300-301) of any preceding claim, wherein the sensor interface is configured to obtain the fetal heart rate signal and/or the uterine activity signal from multiple electrodes, wherein the multiple electrodes are configured for arranging on the abdomen of a pregnant woman.

4.  The device (100, 300-301) of any preceding claim, wherein the processor subsystem is configured to apply a trained uterine activity encoder model to the first and second uterine activity segments to obtain the representations of the first and second uterine activity segments; and to apply a trained fetal heart rate encoder model to the first heart rate segment to obtain the representation of the first fetal heart rate segment.

5.  The device (100, 300-301) of claim 4, wherein

    - a uterine activity segment comprises multiple subsegments, and wherein the processor subsystem is configured to determine a subsegment-by-subsegment encoding of the uterine activity segment using the trained uterine activity encoder model, and/or
    - a fetal heart rate segment comprises multiple subsegments, and wherein the processor subsystem is configured to determine a subsegment-by-subsegment encoding of the fetal heart rate segment using the trained fetal heart rate encoder model.

6.  The device (100, 300-301) of claim 4 or 5, wherein the processor subsystem is further configured to apply a trained fetal heart rate decoder model to the predicted representation of the second fetal heart rate segment to obtain a predicted second fetal heart rate segment, and to output the predicted second fetal heart rate segment.

7.  The device (100, 300-301) of any preceding claim, wherein the processor subsystem is configured to determine anomaly scores at multiple points in time and to aggregate the anomaly scores into an overall anomaly score.

8.  The device (100, 300-301) of any preceding claim, wherein the processor subsystem is configured to raise an alert if the anomaly score and/or the overall anomaly score exceeds a threshold.

9.  The device (100, 300-301) of any preceding claim, wherein the processor subsystem is configured to apply the fetal heart rate prediction model by applying a causal convolutional layer followed by one or more residual blocks.

10. A device (101, 201-205) for training a fetal heart rate anomaly detection, comprising:

- a data interface (121) for accessing a training dataset comprising multiple fetal heart rate signals of fetuses and corresponding uterine activity signals;
- a processor subsystem (131) configured to:

  - using the fetal heart rate signals and corresponding uterine activity signals of the training dataset, train a fetal heart rate prediction model to, based on a representation of a first fetal heart rate segment, a representation of a corresponding first uterine activity segment, and a representation of a subsequent second uterine activity segment, predict a representation of a second fetal heart rate segment;
  - output the fetal heart rate prediction model.

11. The device (101,201,202,204) of claim 10, wherein the processor subsystem is configured to:

    - train a uterine activity autoencoder comprising a uterine activity encoder model and a uterine activity decoder model, and output the trained uterine activity encoder model, and/or
    - train a fetal heart rate autoencoder comprising a fetal heart rate encoder model and a fetal heart rate decoder model, and output the trained fetal heart rate encoder model.

12. The device (101,201,202,204) of claim 11, wherein the processor subsystem is configured to train the fetal heart rate prediction model by applying the fetal heart rate prediction model to obtain a predicted representation of the second fetal heart rate segment; applying the fetal heart rate decoder model to the predicted representation to obtain a predicted fetal heart rate segment; and comparing the predicted fetal heart rate segment to the corresponding fetal heart rate segment of the training dataset.

13. A computer-implemented method (400) of processing a fetal heart rate signal, comprising:

    - accessing (410) a fetal heart rate signal of a fetus and a corresponding uterine activity signal;
    - accessing (420) model data representing a fetal heart rate prediction model configured to, based on an encoding of a first fetal heart rate segment, an encoding of a first uterine activity segment, and an encoding of a second uterine activity segment, predict an encoding of a second fetal heart rate segment;
    - obtaining (430) a first fetal heart rate segment and a subsequent second fetal heart rate segment of the fetal heart rate signal;
    - obtaining (440) a first uterine activity segment and a second uterine activity segment of the uterine activity signal, corresponding respectively to the first and second fetal heart rate segments;
    - applying (450) the fetal heart rate prediction model to a representation of the first fetal heart rate segment, a representation of the first uterine activity segment, and a representation of the second uterine activity segment to obtain a predicted representation of the second fetal heart rate segment;
    - determining (460) an anomaly score indicative of fetal health by comparing the predicted representation of the second fetal heart rate segment to the obtained second fetal heart rate segment, and outputting (470) the anomaly score.

14. A computer-implemented method (500) of training a fetal heart rate anomaly detection, comprising:

    - accessing (510) a training dataset comprising multiple fetal heart rate signals of fetuses and corresponding uterine activity signals;
    - using the fetal heart rate signals and corresponding uterine activity signals of the training dataset, training (520) a fetal heart rate prediction model to, based on a representation of a first fetal heart rate segment, a representation of a corresponding first uterine activity segment, and a representation of a subsequent second uterine activity segment, predict a representation of a second fetal heart rate segment;
    - outputting (530) the trained fetal heart rate prediction model.

15. A computer readable medium (1000) comprising transitory or non-transitory data (1020) representing one or more of:

    - instructions to cause a processor system to perform the method according to claim 13;
    - instructions to cause a processor system to perform the method according to claim 14;
    - a uterine activity encoder model, a fetal heart rate encoder model, and/or fetal heart rate prediction model trained according to the method of claim 14.

100

110

120

130

*Fig. 1a*

101

121

131

*Fig. 1b*

129

*Fig. 1c*

Fig. 2a

Fig. 2b

Fig. 2c

*221* ↘            *222* ↘             <u>*204*</u>

$x_{t-3}^{TOCO}$   $x_{t-2}^{TOCO}$   $x_{t-1}^{TOCO}$   $x_{t}^{TOCO}$   $x_{t+1}^{TOCO}$   $x_{t+2}^{TOCO}$   $x_{t+3}^{TOCO}$   $x_{t+4}^{TOCO}$

$g_{enc}^{TOCO}$   $g_{enc}^{TOCO}$   $g_{enc}^{TOCO}$   $g_{enc}^{TOCO}$   $g_{enc}^{TOCO}$   $g_{enc}^{TOCO}$   $g_{enc}^{TOCO}$   $g_{enc}^{TOCO}$

*230*

$$D_{TOCO} = [\mu_{t-3}^{TOCO}, \ldots, \ldots, \mu_{t+4}^{TOCO}, \sigma_{t-3}^{TOCO} \ldots \sigma_{t+4}^{TOCO}]$$

*231-232* ↗      *260* ↘      $\widehat{D}_{FHR,t+}$    *252'* ↘      *259* ↘

$H_{pred}^{FHR}$

$$D_{FHR,t-} = [\mu^{FHR_{t-3}}, \ldots, \mu^{FHR_t}, \\ \sigma^{FHR_{t-3}}, \ldots, \sigma^{FHR_t}]$$

*251* ↗      *242'* ↘      $g_{dec}^{FHR}$   $g_{dec}^{FHR}$   $g_{dec}^{FHR}$   $g_{dec}^{FHR}$

*250*

$g_{enc}^{FHR}$   $g_{enc}^{FHR}$   $g_{enc}^{FHR}$   $g_{enc}^{FHR}$     $\hat{x}_{t+1}^{FHR}$   $\hat{x}_{t+2}^{FHR}$   $\hat{x}_{t+3}^{FHR}$   $\hat{x}_{t+4}^{FHR}$

$x_{t-3}^{FHR}$   $x_{t-2}^{FHR}$   $x_{t-1}^{FHR}$   $x_{t}^{FHR}$   $x_{t+1}^{FHR}$   $x_{t+2}^{FHR}$   $x_{t+3}^{FHR}$   $x_{t+4}^{FHR}$

*241*                 *242*

**Fig. 2d**

<u>*205*</u>               *260*

*262*

*231*      *261'*          *264*    *266*       *268*

*251*      *261*    *263*          ×    *267*    +

*232*                *265*            *268*

**Fig. 2e**

Fig. 3a

321 ↘    322 ↘    301

$x_{t-3}^{TOCO}$  $x_{t-2}^{TOCO}$  $x_{t-1}^{TOCO}$  $x_t^{TOCO}$  $x_{t+1}^{TOCO}$  $x_{t+2}^{TOCO}$  $x_{t+3}^{TOCO}$  $x_{t+4}^{TOCO}$

$g_{enc}^{TOCO}$  $g_{enc}^{TOCO}$  $g_{enc}^{TOCO}$  $g_{enc}^{TOCO}$  $g_{enc}^{TOCO}$  $g_{enc}^{TOCO}$  $g_{enc}^{TOCO}$  $g_{enc}^{TOCO}$

330

$D_{TOCO}$

331-332

360 ↘    352' ↘    370 ↘    371 ↘

$H_{pred}^{FHR}$    $\widehat{D}_{FHR,t+}$    KLD    $E_{FHR,t+}$

$D_{FHR,t-}$

351    352 ↗    $D_{FHR,t+}$

350

$g_{enc}^{FHR}$  $g_{enc}^{FHR}$  $g_{enc}^{FHR}$  $g_{enc}^{FHR}$  $g_{dec}^{FHR}$  $g_{dec}^{FHR}$  $g_{dec}^{FHR}$  $g_{dec}^{FHR}$

$x_{t-3}^{FHR}$  $x_{t-2}^{FHR}$  $x_{t-1}^{FHR}$  $x_t^{FHR}$  $x_{t+1}^{FHR}$  $x_{t+2}^{FHR}$  $x_{t+3}^{FHR}$  $x_{t+4}^{FHR}$

341    342

*Fig. 3b*

*Fig. 3c*

Fig. 3d

*400*

410

420

430

440

450

460

470

*Fig. 4*

*500*

510

520

530

*Fig. 5*

*1000*

1010

1020

*Fig. 6*

1110

1130

1120

1122

1124

1126

*1140*

*Fig. 7*

Europäisches
Patentamt

European
Patent Office

Office européen
des brevets

# EUROPEAN SEARCH REPORT

Application Number

EP 21 19 3169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/223748 A1 (WARRICK PHILIP A [CA] ET AL) 13 August 2015 (2015-08-13)<br>* paragraph [0015] - paragraph [0017] *<br>* paragraph [0025] - paragraph [0027] *<br>* paragraph [0081] - paragraph [0083] *<br>* paragraph [0087] *<br>* paragraph [0089] - paragraph [0093] *<br>* paragraph [0100] - paragraph [0101] *<br>* paragraph [0104] - paragraph [0106] *<br>* paragraph [0125] *<br>* paragraph [0140] - paragraph [0141] *<br>* figures 1-5,8 * | 1-15 | INV.<br>A61B5/024<br>A61B5/00 |
| A,D | WARRICK P A ET AL: "Classification of Normal and Hypoxic Fetuses From Systems Modeling of Intrapartum Cardiotocography", IEEE TRANSACTIONS ON BIOMEDICAL ENGINEERING, IEEE, USA,<br>vol. 57, no. 4, 1 April 2010 (2010-04-01), pages 771-779, XP011326881, ISSN: 0018-9294, DOI: 10.1109/TBME.2009.2035818<br>* abstract *<br>* page 771, right-hand column, paragraph 5 - page 774, right-hand column, paragraph 6 *<br>* figures 1-5 * | 1-15 | |
| A | US 2005/267376 A1 (MAROSSERO DOROTHEE [US] ET AL) 1 December 2005 (2005-12-01)<br>* paragraph [0020] - paragraph [0022] *<br>* paragraph [0093] *<br>* paragraph [0144] - paragraph [0145] *<br>* figure 3 * | 1-15 | |

TECHNICAL FIELDS
SEARCHED (IPC)

A61B

-/--

The present search report has been drawn up for all claims

| Place of search | Date of completion of the search | Examiner |
|---|---|---|
| Berlin | 8 February 2022 | Völlinger, Martin |

Europäisches
Patentamt
European
Patent Office
Office européen
des brevets

# EUROPEAN SEARCH REPORT

Application Number

EP 21 19 3169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PAUL FERGUS ET AL: "Modelling Segmented Cardiotocography Time-Series Signals Using One-Dimensional Convolutional Neural Networks for the Early Detection of Abnormal Birth Outcomes", ARXIV.ORG, CORNELL UNIVERSITY LIBRARY, 201 OLIN LIBRARY CORNELL UNIVERSITY ITHACA, NY 14853, 6 August 2019 (2019-08-06), XP081457278, * abstract * * page 3, left-hand column, paragraph 3 – page 5, left-hand column, paragraph 1 * * page 9, left-hand column, paragraph 3 * * figures 1,2 * | 1-15 | |
| A | Jinghui Chen ET AL: "Outlier Detection with Autoencoder Ensembles" In: "Proceedings of the 2017 SIAM International Conference on Data Mining", 30 June 2017 (2017-06-30), Society for Industrial and Applied Mathematics, Philadelphia, PA, XP055673679, ISBN: 978-1-61197-497-3 pages 90-98, DOI: 10.1137/1.9781611974973.11, * abstract * * page 5, right-hand column, last paragraph * | 1-15 | TECHNICAL FIELDS SEARCHED (IPC) |

The present search report has been drawn up for all claims

| Place of search | Date of completion of the search | Examiner |
|---|---|---|
| Berlin | 8 February 2022 | Völlinger, Martin |

EPO FORM 1503 03.82 (P04C01)

**ANNEX TO THE EUROPEAN SEARCH REPORT**

**ON EUROPEAN PATENT APPLICATION NO.**                          EP 21 19 3169

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2022

| Patent document cited in search report | | Publication date | Patent family member(s) | | Publication date |
|---|---|---|---|---|---|
| US 2015223748 | A1 | 13-08-2015 | CA | 2918793 A1 | 06-03-2014 |
|  |  |  | US | 2015223748 A1 | 13-08-2015 |
|  |  |  | WO | 2014032160 A1 | 06-03-2014 |
| US 2005267376 | A1 | 01-12-2005 | EP | 1776041 A2 | 25-04-2007 |
|  |  |  | US | 2005267376 A1 | 01-12-2005 |
|  |  |  | US | 2005267377 A1 | 01-12-2005 |
|  |  |  | WO | 2005117692 A2 | 15-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **P. WARRICK et al.** Classification of normal and hypoxic fetuses from systems modeling of intrapartum cardiotocography. *IEEE Transactions on Biomedical Engineering,* 2010, vol. 57 (4), 771-779
- **A. VAN DEN OORD et al.** *WaveNet: A Generative Model for Raw Audio, https://arxiv.org/abs/1609.03499*

- **K. HE et al.** Deep residual learning for image recognition. *Proceedings of the IEEE Computer Society Conference on Computer Vision and Pattern Recognition,* 2016